# EUROPÄISCHE PATENTSCHRIFT

(11) **EP 0 876 337 B1**
(45) Veröffentlichungstag und Bekanntmachung des Hinweises auf die Patenterteilung: **23.02.2005**
(21) Anmeldenummer: 96945986.6
(22) Anmeldetag: 14.11.1996
(51) Int. Cl.: C07C 271/22

(54) **NEUE LH-RH-ANTAGONISTEN MIT VERBESSERTER WIRKUNG**
NEW LH-RH ANTAGONISTS WITH IMPROVED EFFECTIVENESS
NOUVEAUX ANTAGONISTES A EFFICACITE AMELIOREE DE L'HORMONE LIBERANT L'HORMONE LUTEINISANTE

(30) Priorität: 28.11.1995 DE 19544212
(43) Veröffentlichungstag der Anmeldung: 11.11.1998
(73) Patentinhaber: Zentaris GmbH, 60314 Frankfurt/Main (DE)
(72) Erfinder: KUTSCHER, Bernhard, D-63477 Maintal (DE); BERND, Michael, D-60316 Frankfurt (DE); BECKERS, Thomas, D-60596 Frankfurt (DE); KLENNER, Thomas, D-55218 Ingelheim (DE); EMIG, Peter-Paul, D-63486 Bruchköbel (DE); CHARPENTIER, Patricia-Marie, D-63477 Maintal (DE)
(86) Internationale Anmeldenummer: PCT/DE1996/002171
(87) Internationale Veröffentlichungsnummer: WO 1997/019953

(56) Entgegenhaltungen:
- WO-A-94/13313
- US-A- 5 300 492

## Beschreibung

Die Erfindung betrifft neue LH-RH-Antagonisten, insbesondere Peptidomimetika und in einer Seitenkette modifizierte Peptide, Salze derselben mit pharmazeutisch akzeptablen Säuren sowie Verfahren zur Herstellung der LH-RH-Antagonisten und ihrer Salze. Die erfindungsgemäßen Peptide stellen Analoge des das luteinisierende Hormon freisetzenden Hormons (LH-RH) dar, das die folgende Struktur aufweist:
p-Glu-His-Trp-Ser-Tyr-Gly-Leu-Arg-Pro-Gly-NH₂, [LH-RH, Gonadorelin].

Während mehr als 20 Jahren haben Forscher nach selektiv potenten Antagonisten des LH-RH-Dekapeptids [M.Karten und J.E.Rivier, Endocrine Reviews 7, 44-66 (1986)] gesucht. Das hohe Interesse an solchen Antagonisten ist in ihrer Nützlichkeit im Bereich der Endokrinologie, Gynäkologie, Schwangerschaftsverhütung und Krebs begründet. Eine große Anzahl von Verbindungen sind als potentielle LH-RH-Antagonisten hergestellt worden. Die interessantesten Verbindungen, die bis heute gefunden wurden, sind jene Verbindungen, deren Struktur eine Modifizierung der LH-RH-Struktur darstellen.

Die erste Serie von potenten Antagonisten wurde durch die Einführung von aromatischen Aminosäureresten in den Positionen 1, 2, 3 und 6 oder 2, 3 und 6 erhalten. Die übliche Schreibweise der Verbindungen sieht wie folgt aus: es werden zunächst die Aminosäuren angegeben, die in der Peptidkette von LH-RH an die Stelle der ursprünglich vorhandenen Aminosäuren getreten sind, wobei die Positionen, an denen der Austausch stattfand, durch hochgestellte Ziffern gekennzeichnet werden. Weiterhin wird durch die nachgestellte Bezeichnung "LH-RH" zum Ausdruck gebracht, daß es sich um LH-RH-Analoge handelt, an denen der Austausch stattfand.

Bekannte Antagonisten sind:
[Ac-D-Phe(4-Cl)^{1,2}, D-Trp^{3,6}] LH-RH (D,H.Coy et al., In: Gross, E. and Meienhofer, J. (Eds) Peptides; Proceedings of the 6th American Peptid Symposium, S.775-779, Pierce Chem.Co., Rockville III. (1979):
[Ac-Pro¹ ,D-Phe(4-Cl)², D-Nal(2)^{3,6}] LH-RH (US-Patent Nr. 4.419.347) und
[Ac- Pro¹, D-Phe(4-Cl)² ,D-Trp^{3,6}] LH-RH (J.L.Pineda, et al., J. Clin. Endocrinol. Metab. 56, 420, 1983).

Um die Wasserlöslichkeit von Antagonisten zu erhöhen, wurden später basische Aminosäuren, zum Beispiel D-Arg, in der 6-Stellung eingeführt. Zum Beispiel [Ac-D-Phe(4-Cl)^{1,2}, D-Trp³, D-Arg⁶, D-Ala¹⁰] LH-RH (ORG-30276) (D.H.Coy, et al., Endocrinotogy 100, 1445, 1982); und
[Ac-D-Nal(2)¹, D-Phe(4-F)², D-Trp³, D-Arg⁶] LH-RH (ORF 18260) (J.E. Rivier et al., in: Vickery B.H. Nestor, Jr. J.J., Hafez, E.S.E (Eds). LHRH and its Analogs, S.11-22 MTP Press, Lancaster, UK 1984).

Solche Analoge wiesen nicht nur die erwartete verbesserte Wasserlöslichkeit auf, sondern zeigten auch eine verbesserte antagonistische Wirksamkeit. Dennoch verursachen diese äußerst potenten, hydrophilen Analogen mit D-Arg⁶ und anderen basischen Seitenketten an der 6-Stellung vorübergehende Oedeme auf dem Gesicht und den Extremitäten, wenn sie Ratten in Dosen von 1,25 oder 1,5 mg/kg subkutan verabreicht wurden (F.Schmidt, et al., Contraception 29, 283, 1984: J.E. Morgan, et al., Int. Archs. Allergy Appl. Immun. 80, 70 (1986). Weitere potente LH-RH-Antagonisten sind in WO 92/19651, WO 94/19370, WO 92/17025, WO 94/14841, WO 94113313, US-A 5,300,492, US-A 5,140,009 und EP 0 413 209 A1 beschrieben.

Das Auftreten von oedematogenen Auswirkungen nach der Verabreichung von einigen dieser Antagonisten bei Ratten haben Zweifel über ihre Sicherheit bei der Verwendung bei Menschen aufkommen lassen, und so verzögerte sich die Einführung dieser Arzneimittel in die klinische Verwendung. Daher besteht ein großer Bedarf an antagonistischen Peptiden, die frei von Nebenwirkungen sind.

Erfindungsgemäß lösen Verbindungen der allgemeinen Formel (V)

Ac-D-Nal(2)¹-D(pCl)Phe²-D-Pal(3)³-Ser⁴-Tyr⁵-D-Xxx⁶-Leu⁷-Arg⁸-Pro⁹-D-Ala¹⁰-NH₂ (V)

die obengenannte Aufgabe,
wobei D-Xxx eine Aminosäuregruppe der allgemeinen Formel (VI) 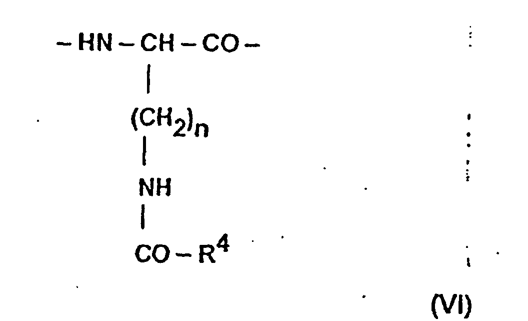 darstellt,
worin n die Zahl 3 oder 4, R⁴ eine Gruppe mit der Formel (II) 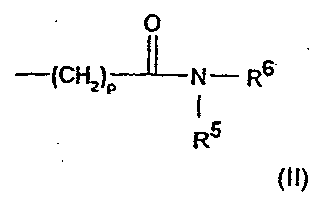 darstellt, in der p eine ganze Zahl von 1 bis 4, R⁵ Wasserstoff oder eine Alkylgruppe und R⁶ eine unsubstituierte oder substituierte Arylgruppe oder Heteroarylgruppe bedeutet,
oder R⁴ einen Ring der allgemeinen Formel (III) 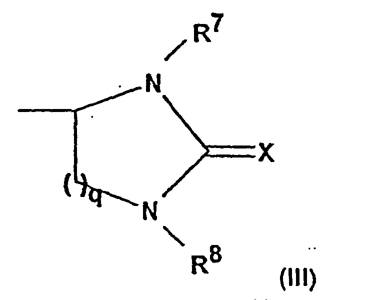 darstellt, in der q die Zahl 1 oder 2, R⁷ ein Wasserstoffatom oder eine Alkylgruppe, R⁸ ein Wasserstoffatom oder eine Alkylgruppe und X ein Sauerstoff- oder Schwefelatom bedeutet, und deren Salze mit pharmazeutisch akzeptablen Säuren.

Bevorzugte Alkylgruppen sind die Methyl-, Ethyl-, n-Propyl-, i-Propyl-, n-Butyl-, i-Butyl-, t-Butyl-, 2-Ethyl-hexyl-, Dodecyl- und Hexadecylgruppe.

Bevorzugte Arylgruppen sind die Phenyl-, Naphthyl-, Phenanthrenyl- und Fluorenylgruppe.

Bevorzugte Heteroarylgruppen sind die 2-, 3- und 4-Pyridyl-, 2- und 4-Pyrimidyl-, Imidazolyl-, Imidazopyridyl-, 5- und 6-Indoiyl-, 5- und 6-Indazolyl-,Triazolyl-, Tetrazolyl-, Benzimidazolyl-, Chinolyl-, 2,6-Dichlor-pyrid-3-yl- und Furylgruppe.

Die erfindungsgemäßen Verbindungen weisen eine hohe antagonistische Potenz auf und sind frei von unerwünschten Nebenwirkungen, insbesondere frei von oedematogenen Wirkungen. Wenn sie nicht als Salze mit schwer wasserlöslichen, pharmazeutisch akzeptablen Säuren vorliegen, weisen sie außerdem eine verbesserte Wasserlöslichkeit auf. Weiterhin sind die Verbindungen hochaffin am humanen LH-RH-Rezeptor, d.h. hochpotent bei der Hemmung der Freisetzung von Gonadotropinen aus der Himanhangdrüse bei Säugetieren, einschließlich des Menschen, weisen langandauemde Testosteronsuppression in Ratten auf und bewirken minimale Histaminfreisetzung in vitro.

Bevorzugte Peptide nach Formel (V) sind solche, bei denen Xxx die [ε-N'-4-(4-Amidino-phenyl)-amino-1,4-dioxo-butyl]-lysyl-Gruppe oder die [ε-N'-(Imidazolidin-2-on-4-yl)-formyl]-lysyl-Gruppe bedeutet. Die Salze mit pharmazeutisch akzeptablen Säuren sind vorzugsweise schwer in Wasser löslich. Besonders bevorzugt sind die Salze der 4,4'-Methylenbis(3-hydroxy-2-naphthoesäure), auch als Embonsäure oder Pamoasäure bekannt.

Die zur Definierung der Peptide verwendete Nomenklatur stimmt mit jener durch die IUPAC-IUB-Kommission über Biochemische Nomenklatur erläuterten Nomenklatur überein (European J.Biochem. 1984, 138, 9-37), worin in Übereinstimmung mit der herkömmlichen Darstellung die Aminogruppen beim N-Terminus nach links erscheinen und die Carboxylgruppe beim C-Terminus nach rechts. Die LH-RH-Antagonisten wie die erfindungsgemäßen Peptide und Peptidomimetika umfassen in der Natur vorkommende und synthetische Aminosäuren, wobei erstere Ala, Val, Leu, Ile, Ser, Thr, Lys, Arg, Asp, Asn, Glu, Gln, Cys, Met, Phe, Tyr, Pro, Trp und His umfassen. Die Abkürzungen für die einzelnen Aminosäurereste beruhen auf den Trivialnamen der Aminosäuren und sind Ala Alanin, Arg Arginin, Gly Glycin, Leu Leucin, Lys Lysin, Pal(3) 3-(3-Pyridyl)alanin, Nal(2) 3-(2-Naphthyl)alanin, Phe Phenylalanin, (pCl)Phe 4-Chlorphenylalanin, Pro Prolin, Ser Serin, Thr Threonin, Trp Tryptophan und Tyr Tyrosin. Alle hier beschriebenen Aminosäuren stammen aus der L-Serie, wenn nicht anders erwähnt. Beispielsweise ist D-Nal(2) die Abkürzung für 3-(2-Naphthyl)-D-Alanin und Ser die Abkürzung für L-Serin. Andere verwendete Abkürzungen sind:
- Boc: tert. Butyloxycarbonyl
- Bop: Benzotriazol-1-yl-oxy-tris-(dimethylamino)-phosphoniumhexafluorophosphat
- DCC: Dicyclohexylcarbodiimid
- DCM: Dichlormethan
- Ddz: Dimethoxyphenyl-dimethylmethylenoxy-carbonyl (Dimethoxydimethyl-Z)
- DIC: Diisopropylcarbodiimid
- DIPEA: N,N-Diisopropylethylamin
- DMF: Dimethylformamid
- Fmoc: Fluorenylmethyloxycarbonyl
- HF: flüssige wasserfreie Flußsäure
- HOBt: 1-Hydroxybenzotriazol
- HPLC: Hochdruckflüssigkeitschromatographie
- PyBop: Benzotriazol-1-yl-oxy-tris-pyrrolidino-phosphoniumhexafluorophosphat
- TFA: Trifluoressigsäure
- Z: Benzyloxycarbonyl

Erfindungsgemäß stellt man Verbindungen der allgemeinen Formel (1) so dar, daß man zunächst zwei der drei Funktionalitäten (α-Amino-, ε-Amino- und α-Carbonsäuregruppe) mit Schutzgruppen versieht und dann die freie dritte Funktionalität in geeigneter Weise umsetzt. Gegebenenfalls kann man auch, wo dies zu besseren Ergebnissen führt, im ersten Schritt intermediäre Schutzgruppen einführen, die man nach dem zweiten Schritt gegen die gewünschte Funktionalität austauscht. Geeignete Schutzgruppen und Verfahren zum Anbringen derselben sind im Fachgebiet bekannt. Beispiele für Schutzgruppen sind in "Principles of Peptide Synthesis", Springer Verlag 1984), im Lehrbuch "Solid Phase Peptide Synthesis" J.M.Stewart and J.D.Young, Pierce Chem.Company, Rockford, III, 1984, und in G.Barany and R.B.Merrifield "The Peptides", Ch.1, S.1-285, 1979, Academic Press Inc. beschrieben.

Die Synthese von Verbindungen gemäß Formel ( V) kann sowohl entweder durch klassische Fragmentkondensation oder per Festphasensynthese nach Merrifield mit aufeinander abfolgendem Aufbau unter Verwendung von in der Seitenkette bereits mit der Carbonsäure der allgemeinen Formel (VII) acyliertem D-Lysin als auch durch Umsetzung eines Decapeptidbausteins mit den entsprechenden Carbonsäuren durch Amid-Verknüpfung in der Seitenkette von D-Lysin⁶ erfolgen. Demnach stehen für das Verfahren zur Herstellung einer Verbindung der allgemeinen Formel (V) erfindungsgemäß drei Alternativen zur Verfügung.

Die erste Möglichkeit umfaßt die Schritte des
(a) Versehens der α-Amino- und der Carbonsäuregruppe von D-Lysin oder D-Omithin mit geeigneten Schutzgruppen,
(b) Umsetzens des mit Schutzgruppen versehenen D-Lysins oder D-Omithins mit einer Carbonsäure der allgemeinen Formel (VII)

   R⁴ - COOH (VII)

   worin R⁴ wie oben definiert ist,
(c) Abspaltens der Schutzgruppe an der α-Carbonsäuregruppe der in Schritt (b) erhaltenen Verbindung zwecks Einbau in Pos. 6 bei Schritt (h),
(d) Ankuppelns von an der Aminogruppe mit einer Schutzgruppe versehenem D-Alanin an einen festen Träger in Form eines Harzes (Merrifield-Synthese),
(e) Abspaltens der Schutzgruppe an der Aminogruppe des Alanins,
(f) Umsetzens des an den festen Träger gebundenen Alanins mit Prolin, das am Stickstoffatom mit einer Schutzgruppe versehen ist,
(g) Abspaltens der Schutzgruppe am Stickstoffatom des Prolins,
(h) Wiederholens der Schritte f) und g) mit den Aminosäuren 1 bis 8 gemäß der allgemeinen Formel (V), in der Reihenfolge von 8 nach 1,unter Verwendung von in Schritt (c) beschriebenem modifiziertem D-Lysin oder D-Omithin für Pos. 6,
(i) Abspaltens der in Schritt (h) erhaltenen Verbindung vom Träger und gegebenenfalls Aufreinigung (z.B. HPLC),
(j) gegebenenfalls Umsetzens mit einer pharmazeutisch akzeptablen Säure, vorzugsweise Embonsäure.

Nach der zweiten Alternative umfaßt das Verfahren zur Herstellung einer Verbindung der allgemeinen Formel (V) die Schritte des
(a) Ankuppelns von an der Aminogruppe mit einer Schutzgruppe versehenem D-Alanin an einen für Festphasensynthese geeigneten Träger,
(b) Abspaltens der Schutzgruppe an der Aminogruppe des Alanins,
(c) Umsetzens des an dem Harz gebundenen Alanins mit Prolin, das am Stickstoffatom mit einer Schutzgruppe versehen ist,
(d) Abspaltens der Schutzgruppe am Stickstoffatom des Prolins.
(e) Wiederholens der Schritte c) und d) mit den Aminosäuren 1 bis 8 gemäß der allgemeinen Formel (V), in der Reihenfolge von 8 nach 1,
(f) Abspaltens der im Schritt (e) erhaltenen Verbindung vom Träger,
(g) Umsetzens mit einer Carbonsäure der Formel (VII)

   R⁴ - COOH (VII)

   worin R⁴ wie oben definiert ist,
(h) gegebenenfalls Umsetzens mit einer pharmazeutisch akzeptablen Säure, vorzugsweise Embonsäure.

Die dritte Variante des Verfahrens zur Herstellung einer Verbindung der allgemeinen Formel (V) umfaßt die Schritte des
(a) Ankuppelns von an der Aminogruppe mit einer Schutzgruppe versehenem D-Alanin an einen Träger, geeignet für Festphasensynthese,
(b) Abspaltens der Schutzgruppe an der Aminogruppe des Alanins,
(c) Umsetzens des an dem Harz gebundenen Alanins mit Prolin, das am Stickstoffatom mit einer Schutzgruppe versehen ist,
(d) Abspaltens der Schutzgruppe am Stickstoffatom des Prolins,
(e) Wiederholens der Schritte c) und d) mit den Aminosäuren 6 bis 8 gemäß der allgemeinen Formel (V), in der Reihenfolge von 8 nach 6,
(f) Abspaltens der ε-Aminoschutzgruppe von D-Lysin oder D-Omithin in Pos. 6 und Umsetzens mit einer Carbonsäure der Formel (VII)

   R⁴ - COOH (VII)

   worin R⁴ wie oben definiert ist,
(g) Abspaltens der Schutzgruppe an der α-Aminogruppe des D-Lysins oder D-Omithins,
(h) Wiederholens der Schritte c) und d) mit den Aminosäuren 1 bis 5 gemäß der allgemeinen Formel (IV), in der Reihenfolge von 5 nach 1,
(i) Abspaltens der in Schritt (h) erhaltenen Verbindung von dem Harz und Aufreinigung (z.B. HPLC),
(j) gegebenenfalls Umsetzens mit einer pharmazeutisch akzeptablen Säure, vorzugsweise Embonsäure.

Bevorzugte Carbonsäuren der allgemeinen Formel (VII) sind Imidazolidin-2-on-4-carbonsäure und N-(4-Amidinophenyl)-amino-4-oxo-buttersäure.

Die Verbindungen der Formel (V) werden nach den bekannten Methoden synthetisiert, wie zum Beispiel durch reine Festphasentechnik, teilweise Festphasentechnik oder durch die klassischen Lösungskupplungen (siehe M.Bodanszky, "Principles of Peptide Synthesis", Springer Verlag 1984).
Zum Beispiel sind die Methoden der Festphasensynthese im Lehrbuch "Solid Phase Peptide Synthesis" J.M.Stewart and J.D.Young, Pierce Chem.Company, Rockford, III, 1984, und in G.Barany and R.B.Merrifield "The Peptides", Ch.1, S.1-285, 1979, Academic Press Inc., beschrieben. Klassische Lösungssynthesen sind ausführlich in der Behandlung "Methoden der Organischen Chemie (Houben-Weyl), Synthese von Peptiden" E.Wünsch (Herausgeber) 1974, Georg Thieme Verlag, Stuttgart, BRD, beschrieben.

Der stufenweise Aufbau erfolgt zum Beispiel, indem man zunächst die Carboxy-terminate Aminosäure, deren α-ständige Aminogruppe geschützt ist, an einen hierfür üblichen unlöslichen Träger kovalent bindet, die α-Amino-Schutzgruppe dieser Aminosäure abspaltet, an die so erhaltene freie Aminogruppe die nächste geschützte Aminosäure über ihre Carboxy-Gruppe bindet, und in dieser Weise Schritt für Schritt die übrigen Aminosäuren des zu synthetisierenden Peptids in der richtigen Reihenfolge verknüpft, und nach Verknüpfung aller Aminosäuren das fertige Peptid vom Träger abspaltet und gegebenenfalls weitere vorhandene Seitenfunktions-Schutzgruppen abspaltet. Die stufenweise Kondensation erfolgt durch Synthese aus den entsprechenden, in üblicher Weise geschützten Aminosäuren in herkömmlicher Weise. Ebenfalls ist die Verwendung automatischer Peptid-Synthesizer, zum Beispiel Typ Labortec SP 650 von Fa. Bachem, Schweiz, möglich unter Verwendung der im Handel erhältlichen geschützten Aminosäuren.

Die Verknüpfung der einzelnen Aminosäuren miteinander erfolgt nach den hierfür üblichen Methoden, insbesondere kommen in Frage:
- Methode der symmetrischen Anhydride in Gegenwart von Dicyclohexylcarbodiimid oder Diisopropylcarbodiimid (DCC, DIC)
- Carbodiimid-Methode allgemein
- Carbodiimid-Hydroxybenzotriazol-Methode
(siehe The Peptides, Volume 2, Ed. E. Gross and J. Meienhofer). Für die Verknüpfung von Arginin wird vorzugsweise die Carbodiimid-Methode benutzt. Für die übrigen Aminosäuren wird im allgemeinen die Methode der symmetrischen oder gemischten Anhydride benutzt.

Bei der Fragmentkupplung verwendet man vorzugsweise die ohne Racemisierung verlaufende Azidkupplung oder die DCC-1-Hydroxybenzotriazol- beziehungsweise DCC-3-Hydroxy-4-oxo-3,4-dihydro-1,2,3-benzotriazin-Methode. Man kann auch aktivierte Ester von Fragmenten einsetzen.

Für die stufenweise Kondensation von Aminosäuren eignen sich besonders gut aktivierte Ester von N-geschützten Aminosäuren, wie zum Beispiel N-Hydroxysuccinimidester oder 2,4,5-Trichlorphenylester. Die Aminolyse läßt sich sehr gut durch N-Hydroxyverbindungen, die in etwa die Acidität der Essigsäure besitzen, wie zum Beispiel 1-Hydroxybenzotriazol, katalysieren.

Als intermediäre Aminoschutzgruppen bieten sich abhydrierbare Gruppen, wie zum Beispiel der Benzyloxycarbonylrest (= Z-Rest) oder schwach sauer abspaltbare Gruppen an. Als Schutzgruppen für die α-ständigen Aminogruppen kommen zum Beispiel in Frage:
tertiäre Butyloxycarbonylgruppen, Carbobenzoxygruppen beziehungsweise Carbobenzthiogruppen (gegebenenfalls jeweils mit p-Brom oder p-Nitrobenzylrest), die Trifluoracetylgruppe, der Phthalylrest, die o-Nitrophenoxyacetylgruppe, die Tritylgruppe, die p-Toluolsulfonylgruppe, die Benzylgruppe, im Benzolkem substituierte Benzylreste (p-Brom oder p-Nitrobenzylrest) und der α-Phenyl-ethylrest. Hierzu wird auch auf das Buch von Jesse P. Greenstein und Milton Winitz, Chemistry of Amino Acids, New York 1961, John Witey and Sons, Inc., Volume 2, beispielsweise Seite 883 und folgende sowie The Peptides, Volume 2, Ed.E. Gross and J. Meienhofer, Academic Press, New York, verwiesen. Diese Schutzgruppen kommen grundsätzlich auch für den Schutz von weiteren funktionellen Seitengruppen (OH-Gruppen, NH₂-Gruppen) der entsprechenden Aminosäuren in Frage.

Vorhandene Hydroxygruppen (Serin, Threonin) werden vorzugsweise durch Benzylgruppen und ähnliche Gruppen geschützt. Weitere nicht α-ständige Aminogruppen (zum Beispiel Aminogruppen in ω-Stellung, Guanidinogruppe des Arginins) werden vorzugsweise orthogonal geschützt.

Die Reaktion zur Verknüpfung vom Aminosäuren findet in einem hierfür üblichen indifferenten Lösungs- oder Suspensionsmittel (zum Beispiel Dichlormethan) statt, wobei gegebenenfalls zur Verbesserung der Löslichkeit Dimethylformamid zugesetzt werden kann.

Für die Einführung der R⁴-CO-Gruppe durch Umsetzen der Aminogruppe des Lysins mit der Carbonsäure der allgemeinen Formel (VII) kommen grundsätzlich die gleichen Verfahren wie oben für die Verknüpfung der Aminosäuren beschriebenen in Frage. Besonders bevorzugt ist jedoch die Kondensation unter Verwendung von Carbodiimid, beispielsweise 1-Ethyl-3-(3-dimethylaminopropyl)-carbodiimid, und 1-Hydroxybenzotriazol.

Als synthetisches Trägermaterial kommen unlösliche Polymere in Frage, zum Beispiel in organischen Lösungsmitteln quellbares Polystyrolharz in Perlenform (beispielsweise ein Copolymerisat aus Polystyrol und 1% Divinylbenzol). Der Aufbau eines geschützten Decapeptidamids an einem Methyl-benzhydrylamid-Harz (MBHA-Harz, d.h. mit Methyl-benzhydrylamid-Gruppen versehenes Polystyrolharz), welches die gewünschte C-terminale Amidfunktion des Peptids nach HF-Spaltung vom Träger ergibt, kann gemäß folgendem Fließdiagramm durchgeführt werden:

### Fließdiagramm

### Peptid-Syntheseprotokoll

| **Stufe** | **Funktion** | **lösungsmittel/Reagenz (v/v)** | **Zeit** |
|---|---|---|---|
| 1 | Waschen | Methanol | 2 x 2 min |
| 2 | Waschen | DCM | 3 x 3 min |
| 3 | Abspaltung | DCM/TFA (1:1) | 1 x 30 min |
| 4 | Waschen | Isopropanol | 2 x 2 min |
| 5 | Waschen | Methanol | 2 x 2 min |
| 6 | Waschen | DCM | 2 x 3 min |
| 7 | Neutralisation | DCM/DIPEA (9:1) | 3 x 5 min |
| 8 | Waschen | Methanol | 2 x 2 min |
| 9 | Waschen | DCM | 3 x 3 min |
| 10 | STOP | Zugabe der Boc-As in DCM + DIC + HOBt | |
| 11 | Kupplung | - | ca. 90 min |
| 12 | Waschen | Methanol | 3 x 2 min |
| 13 | Waschen | DCM | 2 x 3 min |

Die N-α-Boc-geschützten Aminosäuren werden in dreifachem molaren Überschuß in Gegenwart von Diisopropylcarbodiimid (DIC) und 1-Hydroxybenzotriazol (HOBt) in CH₂Cl₂/DMF innerhalb von 90 min. gekuppelt, und die Boc-Schutzgruppe durch halbstündige Einwirkung von 50% Trifluoressigsäure (TFA) in CH₂Cl₂ abgespalten. Zur Kontrolle des vollständigen Umsatzes kann der Chloraniltest nach Christensen und der Kaiser'sche Ninhydrintest dienen. Reste freier Aminofunktion werden durch Acetylierung in fünffachem Überschuß an Acetylimidazol in CH₂Cl₂ blockiert.

Die Abfolge der Reaktionsschritte des Peptidaufbaus am Harz ergibt sich aus dem Fließdiagramm. Zur Abspaltung der harzgebundenen Peptide wird das jeweilige Endprodukt der Festphasensynthese im Vakuum über P₂O₅ getrocknet und in 500fachem Überschuß an HF/Anisol 10:1/V:V 60 min. bei 0°C behandelt.

Nach Abdestillation von HF und Anisol im Vakuum fallen die Peptidamide durch Ausrühren mit wasserfreiem Ethylether als weiße Feststoffe an, die Abtrennung von mit anfallendem polymeren Träger erfolgt durch Auswaschen mit 50%iger wäßriger Essigsäure. Durch schonendes Einengen der essigsauren Lösungen im Vakuum können die jeweiligen Peptide als hochviskose Öle erhalten werden, welche sich nach Zugabe von abs.Ether in der Kälte in weiße Feststoffe umwandeln.

Die weitere Aufreinigung erfolgt durch Routinemethoden der präparativen Hochdruck-Ftüssigskeitschromatographie (HPLC).

Das Überführen der Peptide in ihre Säureadditionssalze kann durch Umsetzen derselben mit Säuren in an sich bekannter Weise bewerkstelligt werden. Umgekehrt können freien Peptide durch Umsetzen ihrer Säureadditionssalze mit Basen erhalten werden. Peptidembonate können durch Umsetzung von Trifluoressigsäuresatzen (TFA-Salzen) des Peptids mit freier Embonsäure (Pamoasäure) oder dem entsprechenden Dinatrium-Salz der Embonsäure dargestellt werden. Dazu wird das Peptid-TFA-Salz in wäßriger Lösung mit der Lösung von Dinatrium-embonat in dipolar-aprotischem Medium, bevorzugt Dimethylacetamid, versetzt und der sich bildende hellgelbe Niederschlag isoliert.

Die folgenden Beispiele erläutern die Erfindung, ohne sie zu beschränken.

### Beispiel 1

### Ac-D-Nal(2)-D(pCl)Phe-D-Pal(3)-Ser-Tyr-D-[ε-N'-(Imidazolidin-2-on-4-yl)-formyl]-Lys-Leu-Arg-Pro-D-Ala-NH₂

Die Synthese erfolgte gemäß Fließdiagramm an 5 g mBHA-Harz (Beladungsdichte 1.08 mmol/g). Lysin wurde als Fmoc-D-Lys(Boc)-OH gekuppelt und nach Abspaltung der Boc-Gruppe in der Seitenkette mit lmidazolidin-2-on-4-carbonsäure im 3-fachen Überschuß acyliert. Nach Abspaltung der Fmoc-Schutzgruppe mit 20% Piperidin/DMF wurde gem. Fließdiagramm zum N-Terminus verlängert. Nach Abspaltung vom polymeren Träger fielen 5,2 g Rohpeptid an, welches durch Standardverfahren der präparativen HPLC aufgereinigt wurden. Nach anschließender Gefriertrocknung erhielt man 2,1 g HPLC-einheitliches Produkt der Summenformel C₇₄H₉₇N₁₈O₁₅Cl mit korrektem FAB-MS 1514 (M+H⁺) (ber.1512,7) und entsprechendem ¹H-NMR-Spektrum.
¹H-NMR (500 MHz, DMSO-d₆, δ in ppm):
8.56, m, 2H, arom.H; 8,08, m, 1 H, arom.H; 7,81, m, 1 H, arom. H; 7,73 m, 2H, arom.H; 7,66, m, 1 H, arom.H; 7,60, s, 1H, arom.H; 7,44, m, 2H, arom.H; 7,30, d, 1 H, arom.H; 7,25, u. 7,18, 2d, 2x2H, arom.H p-CI-Phe; 6,97 u. 6,60, 2d, 2x2H, arom.H Tyr; 9,2-6,3, mehrere Signale, Amid-NH; 4,8-4,0, mehrere m, Cα-H u. aliph.H; 2,1-1,1, mehrere m, restl.aliphat.H; 1,70, s, 3H, Acetyl; 1,22, d, 3H, Cβ-H Ala; 0,85, dd, 6H, Cδ-H Leu

### Beispiel 2

### Ac-D-Nal(2)-D(pCl)Phe-D-Pal(3)-Ser-Tyr-D-[ε-N'-4-(4-Amidino-phenyl)-amino-1,4-dioxo-butyl]-Lys-Leu-Arg-Pro-D-Ala-NH₂

0,7 mmol (1,03 g) Decapeptid Ac-D-Nal-D-(pCl)Phe-D-Pal-Ser-Tyr-D-Lys-Leu-Arg-Pro-D-Ala-NH₂ wurden mit 1,0 mmol (0,27 g) (4-Amidinophenyl)amino-4-oxo-buttersäure in Gegenwart von 1,0 mmol (0,16 g) 1-Ethyl-3-(3-dimethylaminopropyl)-carbodiimid und 1,0 mmol (0,16 g) 1-Hydroxybenzotriazol in frisch destilliertem DMF umgesetzt. Das Lösungsmittel wurde nach 24h im Vakuum entfernt, der anfallende Rückstand in Wasser gelöst und gefriergetrocknet. Das angefallene rohe Reaktionsprodukt (1,63 g) wurde durch präparative Reverse-Phase HPLC aufgereinigt; insgesamt fielen 0,61 g HPLC-einheitliches Produkt der Summenformel C₈₁H₁₀₄N₁₉O₁₅Cl mit korrektem FAB-MS: 1618,7 (M+H⁺) (ber. 1617,7) und entsprechendem ¹H-NMR-Spektrum an.
¹H-NMR (500 MHz, DMSO-d₆, δ in ppm):
10,4, s, 1 H u. 9,15, s, 2H, u. 8,8, s, 1H, NH's von 4-Amidinoanilin; 8,60, m, 2H, arom.H; 8,20, m, 1 H, arom.H; 7,80, m, 1H, arom.H; 7,73, m, arom.H; 7,61, s, 1H, arom.H; 7,44, m, 2H, arom.H; 7,30, d,1H, arom.H; 7,25 u. 7,20, 2d, 4H, arom.H (pCl)Phe; 7,0 u. 6,6, 2d, 4H, arom.H Tyr; 8,3 - 7,2, mehrere Signale, Amid-NH; 4,73 - 4,2, mehrere Multipletts, Cα-H; 4,13, m, 1H, Cα-H; Ala; 3,78-2,4, mehrere Multipletts, Cβ-H und aliphat.H; 1,72, s, 3H, Acetyl; 1,22, d, 3H, Cβ Ala; 0,85, dd, 6H, Cδ Leu

### Beispiel 3

0,5 g (0,3 mmol) peptidischer LH-RH-Antagonist gem. Beispiel 1, gelöst in 50 ml H₂O, wurde durch Reaktion mit 0,130 g (0,3 mmol) Embonsäure-Dinatriumsalz in 2 ml wäßriger Lösung zu Peptid-Embonat umgesetzt, welches sich als gelbes Präzipitat rasch aus der Lösung abschied. Man erhielt 0,281 g feinkristallines gelb-grünes Pulver, Embonsäuregehalt 33%.

### Beispiel 4

0,3 g (0,17 mmol) peptidischer LH-RH-Antagonist gem. Beispiel 2, gelöst in 5 ml Dimethylacetamid, wurde durch Reaktion mit 0,195 g (0,45 mmol) Embonsäure-Dinatriumsalz in 2 ml wäßriger Lösung zu Peptid-Embonat umgesetzt, welches nach Zugabe von 50 ml H₂O als gelber Niederschlag anfiel. Man erhielt 0,330 g feinkristallines gelbes Produkt, Embonsäuregehalt 20%.

### Beispiel 5 Bindungsaffinitäten von Cetrorelix, Beispiel 1 und Beispiel 2 am humanen LH-RH-Rezeptor

### Cetrorelix: Ac-D-Nal(2)-D-p-Cl-Phe-D-Pal(3)-Ser-Tyr-D-Cit-Leu-Arg-Pro-D-Ala-NH₂)

Methode zur Bestimmung der Bindungsaffinität (Dissoziationskonstanten Kd): Die Bestimmung der Bindungsaffinität erfolgt durch einen Kompetitions-Bindungstest ("displacement binding experiment"; Beckers et al. Eur. J. Biochem. 231, 535-543, 1995). Verwendet wird als radioaktiv markierter Ligand [¹²⁵J] Cetrorelix (spezifische Aktivität 5-10x10⁵ dpm/pmol; gelöst in 20% v:v Acetonitril, 0.2 % w:v Albumin, 0.1 % w:v TFA, ≈80% v:v Aqua). Die Bindungsfähigkeit des iodierten Peptides beträgt zwischen 60% und 85%. Als nicht markierte Testverbindungen werden Cetrorelix, Beispiel 1, Beispiel 2 in Lösung verwendet. Die Substanzen werden in den Konzentrationen von 0.01nM - 1000nM (Cetrorelix, Beispiel 1, Beispiel 2) eingesetzt.

Die für den Bindungstest verwendeten Zellen des den humanen LH-RH-Rezeptor überexprimierenden Einzetzellklons L3.5/78 werden mit PBS/EDTA (PBS ohne Ca²⁺/Mg²⁺ / 1mM EDTA) von einer nicht konfluenten bewachsenen Zellkulturschale abgelöst, die Zellzahl bestimmt und in einer entsprechenden Zelldichte in Inkubationsmedium (Dulbecco's modified Eagle Medium mit 4.5g/l Glucose, 10mM Hepes pH7.5, 0.5% w:v BSA, 1g/l Bacitracin, 0.1g/l SBTI, 0.1% w:v NaN₃) resuspendiert. In spezielle 400µl Reaktionsgefäße (Fa. Renner Typ Beckman) legt man 200µl Silicon/Paraffinöl Mischung (84 / 16vol%) vor und pipettiert 50µl der Zellsuspension (2,5x10⁵ Zellen) darauf. Zur Zellsuspension auf der Silicon/Paraffinöl Schicht werden dann sofort 50µl Bindungsmedium mit [¹²⁵J] Cetrorelix und der zu testenden Verbindung in der entsprechenden Konzentration zugegeben. Nun wird unter Drehen in einem Wärmeschrank bei 37°C für 60min inkubiert. Nach diesem Schritt wird in der Heraeus Biofuge 15 im Rotor HTA 13.8 für 2min bei 9000upm (Raumtemperatur) zentrifugiert. Die Zellen pelletieren dabei durch die Silicon/Paraffinöl Schicht und werden so vom Bindungsmedium getrennt. Nach der Zentrifugation werden die Reaktionsgefäße in flüssigem N₂ schockgefroren und die Spitze des Reaktionsgefäßes (Zellpellet) mit einer Zange abgeschnitten und die Spitze mit dem Zellpellet (gebundener Ligand [¹²⁵J] Cetrorelix) als auch der Überstand dem Zellpellet (gebundener Ligand [¹²⁵J] Cetrorelix) als auch der Überstand (nicht gebunder, freier Ligand [¹²⁵J] Cetrorelix) in Zählröhrchen überführt. Zur Bestimmung der maximalen Bindung (Bo) wird kein Kompetitor zugegeben. Für die Bestimmung der unspezifischen Bindung wird 1µM nicht markiertes Cetrorelix zur Kompetition zugegeben. Die unspezifische Bindung ist mit ≤ 10% der Gesamtbindung Bo niedrig. Die Quantifizierung erfolgt in einem γ-Counter, die Auswertung erfolgt mit dem Programm EBDA/Ligand V3.0 (McPherson, J. Pharmacol. Methods 14, 213-228, 1985). Die Auftragung im Dosis-Wirkung-Diagramm ermöglicht die Abschätzung der IC₅₀ (Konzentration, die 50% Inhibierung der Reaktion am Rezeptor bewirkt), das Programm EBDA/Ligand berechnet hieraus die Dissoziationskonstante Kd [nM].

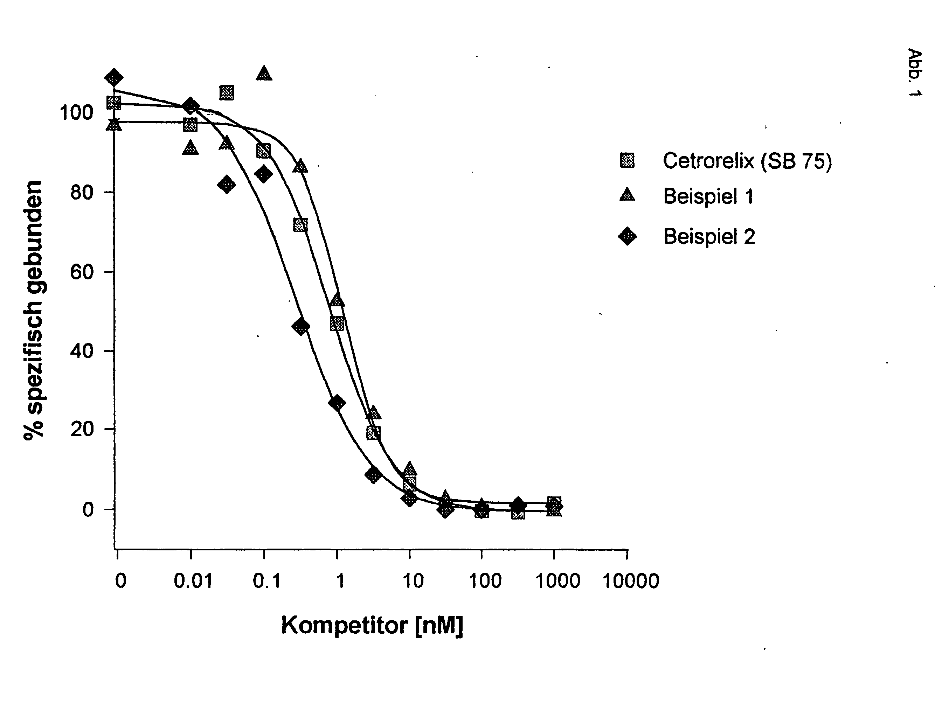

Ergebnis: Aus den Kompetitionskurven (siehe Abb. 1) wird ersichtlich, daß alle getesteten Verbindungen mit dem radioaktiv markierten Ligand [¹²⁵J] Cetrorelix) um die Bindung am humanen LH-RH-Rezeptor kompetitieren. Aufgetragen ist jeweils die Bindung (in % der Gesamtbindung Bo) gegen die Konzentration des Kompetitors. Für die in der Abb. 1 gezeigten Verbindungen konnten folgende Bindungsaffinitäten berechnet als Dissoziationskonstante Kd [nM] berechnet werden: Cetrorelix (SB-75): 0.214 nM, Beispiel 1: 0.305 nM, Beispiel 2: 0.104nM,

Die Bindungsaffinitäten als Mittelwert verschiedener Bestimmungen sind aus Tabelle 1 zu entnehmen.

### Beispiel 6 Antagonistische Wirkung von Beispiel 2 im funktionalen Assay am humanen LH-RH Rezeptor

Methode zur Bestimmung von IP₃ (D-myo-1,3,5-trisphosphat): Eine subkonfluente Kultur des den humanen LH-RH-Rezeptor überexprimierenden Zellklons (L 3.5/78) wird 1x mit PBS gewaschen, die Zellen mit PBS/EDTA abgelöst und die Zellsuspension pelletiert. Die Zellen werden in Inkubationsmedium (Dulbecco's modified eagle Medium mit 4.5g/l Glucose, 10mM Hepes pH7.5, 0.5% w:v BSA, 5mM LiCl, 1g/l Bacitracin, 0.1g/l SBTI) resuspendiert, in 1.5 ml Reaktionsgefäßen aliquotiert und für 30min bei 37°C vorinkubiert. Benötigt werden 4x10⁶ Zellen in 500µl Volumen je Meßpunkt. Nach dem Vorinkubationsschritt erfolgt die Zugabe von LH-RH (Stammlösung 0.5mM in 10mM Tris pH 7.5, 1 mM Dithiothreitol, 0.1 % w:v BSA/Bachem Art # H4005) zur Zellsuspension in einer Endkonzentration von 10nM. Die Wirkung eines Antagonisten wird durch gleichzeitige Zugabe in der entsprechenden Konzentration (beispielsweise 0.0316, 0.1, 0.316 etc. bis 100nM für Beispiel 2) getestet. Als Negativkontrolle werden Zellen ohne zugegebenes LH-RH inkubiert. Nach 15min Inkubation bei 37°C wird gebildetes IP₃ aus den Zellen mittels Trichloressigsäure (TCA)- Extraktion isoliert. Hierzu gibt man 500µl eiskalte 15% (w:v) TCA-Lösung zur Zellsuspension zu. Das entstehende Präzipitat wird durch Zentrifugation bei 4°C, bei 2000xg für 15min in der Heraeus Biofuge 15R pelletiert. Der Überstand von 950µl wird 3x mit 10Vol kaltem, wassergesättigtem Diethylether in einem 15ml Gefäß auf Eis stehend extrahiert. Nach dem letzten Extraktionsschritt wird die Lösung mit 0.5M NaHCO₃-Lösung auf einen pH Wert von 7.5 eingestellt.

Die Bestimmung der IP₃ Konzentration in den Zellextrakten wird mittels eines sensitiven kompetiven Bindungstest mit einem IP₃-Bindungsprotein, markiertem [³H]-IP₃ und nicht markiertem IP₃ durchgeführt. Hierzu wird ein Assay Kit von Amersham (TRK 1000) verwendet; die Durchführung erfolgt wie in dem Assay-Protokoll beschrieben. Nach Durchführung der verschiedenen Schritte wird zuletzt 2ml Szintillator für wässrige Proben (Rotiszint Ecoplus) zugegeben, das resuspendierte Pellet mit dem gebundenen [³H]-IP₃ hiermit sorgfältig gemischt, und in einem β-Szintillationszähler gemessen. Die Menge an zellulärem IP₃ wird unter Verwendung einer Standardkurve berechnet und eine Dosis-Wirkungskurve erstellt. Die IC₅₀ kann aus dem Wendepunkt dieser Kurve abgeschätzt werden.

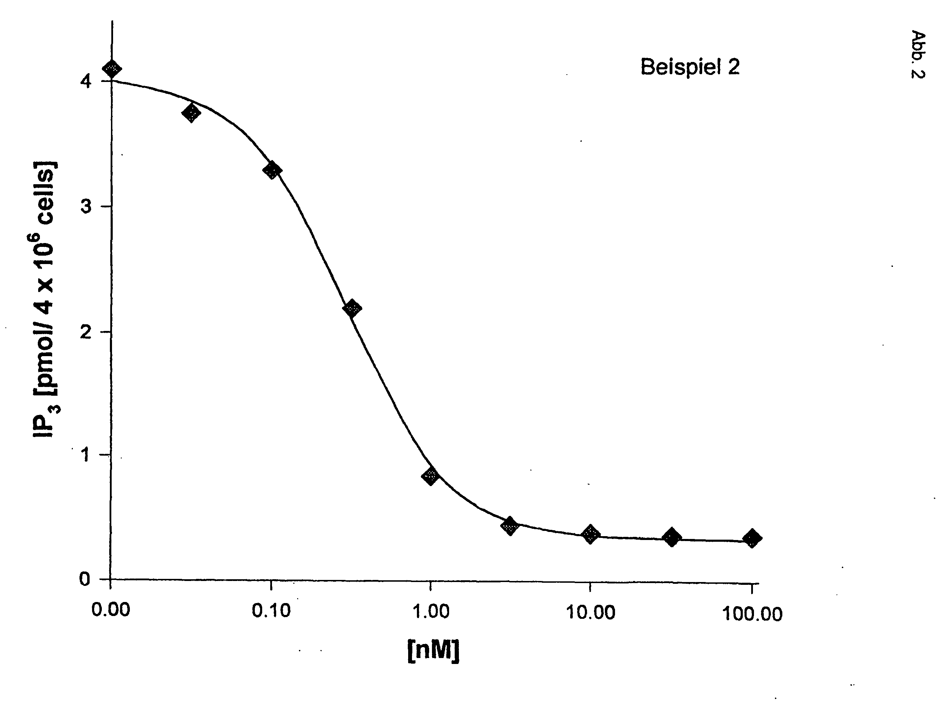

Ergebnis: In der Abb. 1 sind entsprechende Dosis-Wirkungskurven für den peptidischen Antagonisten Beispiel 2 (Abb. 2) dargestellt. Stimuliert wurde mit 10nM LH-RH und die Hemmung der Bildung von IP₃ in Abhängigkeit von der Substanz-Konzentration bestimmt. Für Beispiel 2 konnte keine agonistische Aktivität bestimmt werden, d.h. die Substanzen alleine führen nicht zu einer Stimulierung der IP₃-Synthese. In hier nicht dargestellten Kontrollexperimenten wurde gezeigt, daß nicht-transfektierte Zellen durch LH-RH nicht zur IP₃ - Synthese stimuliert werden können. Die mit den höchsten Konzentrationen noch meßbaren IP₃-Konzentrationen entsprechen denen von unstimulierten Zellen. Es handelt sich bei Beispiel 2 somit um funktionale Antagonisten von LH-RH. Die Substanzen unterscheiden sich allerdings in Ihrer Potenz. Unter den gewählten Versuchsbedingungen ist die IC₅₀ von Beispiel 2 bei etwa 0.4nM.
Diese Aktivitäten korrelieren sehr gut mit den in-vitro im Kompetitions-Bindungstest mit [¹²⁵J]- Cetrorelix bestimmten Bindungsaffinitäten von Kd = 0,109 nM für Beispiel 2.

### Beispiel 7 Hormonsuppressive Wirkung von Beispiel 1, Beispiel 2 bei der gesunden männlichen Ratte

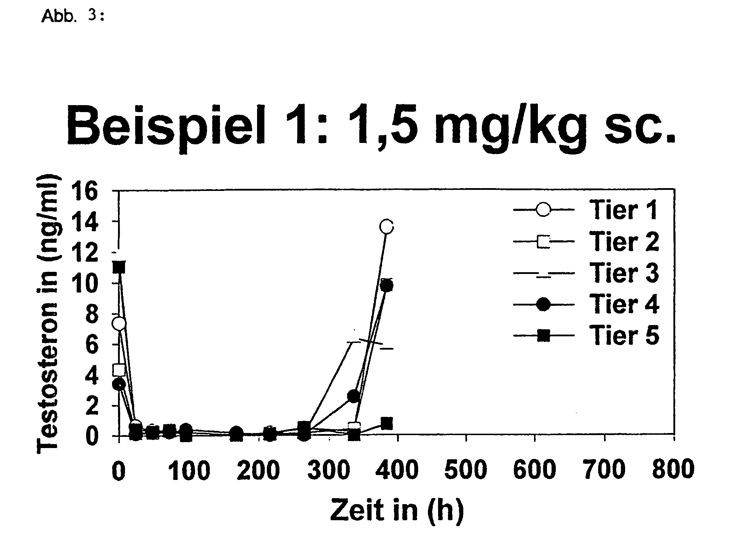

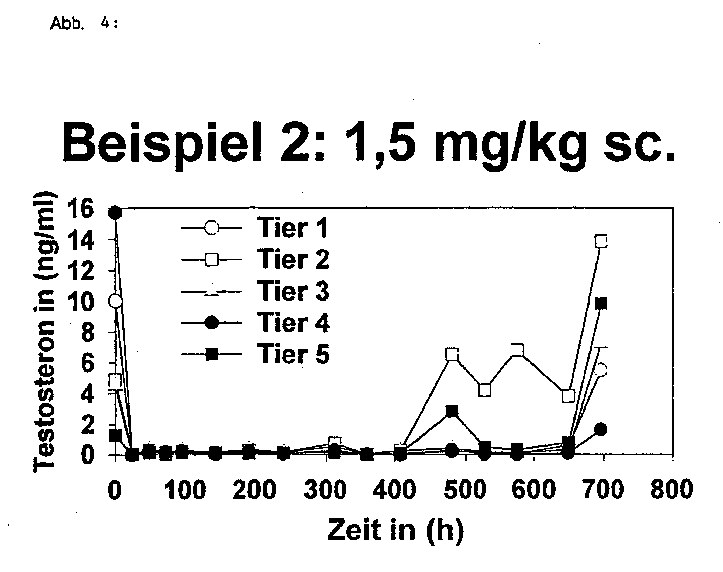

Zur Bestimmung der Suppression von Testosteron im Blut gesunder männlicher Ratten wurden den Tieren die Substanz subkutan in die rechte Flanke injiziert. Die Dosierung betrug bei Beispiel 1 und Beispiel 2 1.5 mg/kg. Zur Kontrolle der Testosteronwerte wurde an den Zeitpunkten 0, 2, 4, 24, 48, 72, 96 Stunden, und dann alle 3 Tage bis zum Suppressionsende den Tieren ca. 300 µl Blut aus der Vena sublingualis entnommen. Die Suppression unter 1 ng/ml Testosteron hielt nach der Gabe von Beispiel 1 bei einem Tier bis 264 Stunden, bei zwei Tieren bis 336 Stunden und bei einem Tier bis 384 Stunden an (Abb.3 ). Nach Gabe von Beispiel 2 wurde der Testosteronspiegel bei einem Tier bis 408 Stunden , bei vier Tieren bis 648 Stunden supprimiert (Abb. 4).

**Tabelle 1:**

| Biologische Daten | | | | |
|---|---|---|---|---|
| Bindungsaffinitäten am humanen LH-RH-Rezeptor (ausgedrückt als Dissoziationskonstante Kd [nM]; Auswertung mit dem EBDA/Ligand Analyse Programm. Angegeben sind Mittelwerte aus verschiedenen Experimenten, Anzahl der Experimente in Klammem) sowie Testosteronsuppression in vivo, Histaminrelease in vitro und Wasserlöslichkeit im Vergleich zu SB-75: | | | | |

| Substanz | Affinität humaner LH-RH-Rezept. [nmol/L] | (1,5 mg/kg, Einmalgabe) Testosteronsuppr. Ratten [h] | (IC₅₀) Histamin-release [µg/ml] | H₂O-Löslichkeit [mg/ml] |
|---|---|---|---|---|
| Cetrorelix SB-75 | 0,202 (10) | 144 | 9,7 | 9 |
| Beispiel 1 | 0.306 (2) | 336 | 31,9 | 27 |
| Beispiel 2 | 0,109(2) | 648 | 17,1 | 23 |
| Beispiel 3 | 0,170 (2) | 864 | n.b.* | n.b. |
| Beispiel 4 | 0,206 (2) | 696 | n.b. | n.b. |

| | | | | |
|---|---|---|---|---|
| *)wg. Schwerlöslichkeit nicht bestimmbar | | | | |

## Patentansprüche

1. Verbindung der allgemeinen Formel V
Ac-D-Nal(2)¹-D(pCl)Phe²-D-Pal(3)³-Ser⁴-Tyr⁵-D-Xxx⁶-Leu⁷-Arg⁸-Pro⁹-D-Ala¹⁰-NH₂ (V)
worin D-Xxx eine Aminosäuregruppe der allgemeinen Formel (VI) 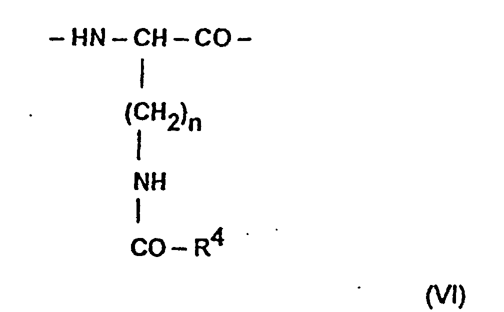 darstellt,
worin n die Zahl 3 oder 4, R⁴ eine Gruppe mit der Formel (II) 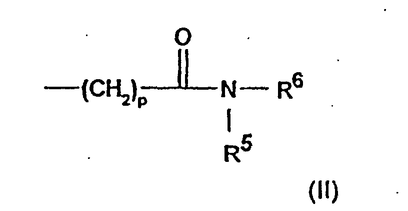 darstellt, in der p eine ganze Zahl von 1 bis 4, R⁵ Wasserstoff oder eine Alkylgruppe und R⁶ eine unsubstituierte oder substituierte Arylgruppe oder
Heteroarylgruppe bedeutet,
oder R⁴ einen Ring der allgemeinen Formel (III) 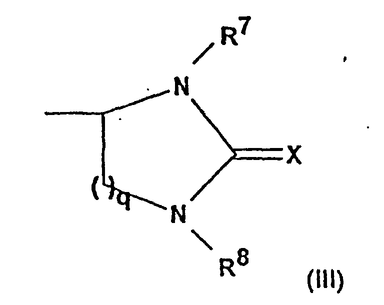 darstellt, in der q die Zahl 1 oder 2, R⁷ ein Wasserstoffatom oder eine Alkylgruppe, R⁸ ein Wasserstoffatom oder eine Alkylgruppe und X ein Sauerstoff- oder Schwefelatom bedeutet, und deren Salze mit pharmazeutisch akzeptablen Säuren.

2. Verbindung nach Anspruch 1, worin Xxx eine [ε-N-4-(4-Amidino-phenyl)-amino-1,4-dioxo-butyl]-lysyl-Gruppe bedeutet.

3. Verbindung nach Anspruch 1, worin Xxx eine [ε-N-(Imidazolidin-2-on-4-yl)-formyl]-lysyl-Gruppe bedeutet.

4. Verbindung nach einem der Ansprüche 1 bis 3, worin das Salz ein Embonat ist.

5. Pharmazeutische Zusammensetzung, umfassend eine Verbindung nach einem der Ansprüche 1 bis 4.

6. Verfahren zur Herstellung einer Verbindung nach Anspruch 1, umfassend die Schritte des
(a) Versehens der α-Amino- und der Carbonsäuregruppe von D-Lysin oder D-Omithin mit geeigneten Schutzgruppen,
(b) Umsetzens des mit Schutzgruppen versehenen D-Lysins oder D-Omithins mit einer Carbonsäure der allgemeinen Formel (VII)
R⁴ - COOH (VII)
worin R⁴ wie in Anspruch 1 definiert ist,
(c) Abspaltens der Schutzgruppe an der α-Carbonsäuregruppe der in Schritt (b) erhaltenen Verbindung zwecks Einbau in Pos. 6 bei Schritt (h),
(d) Ankuppeins von an der Aminogruppe mit einer Schutzgruppe versehenem D-Alanin an einen festen Träger in Form eines Harzes.
(e) Abspaltens der Schutzgruppe an der Aminogruppe des Alanins,
(f) Umsetzens des an den festen Träger gebundenen Alanins mit Prolin, das am Stickstoffatom mit einer Schutzgruppe versehen ist,
(g) Abspaltens der Schutzgruppe am Stickstoffatom des Prolins,
(h) Wiederholens der Schritte f) und g) mit den Aminosäuren 1 bis 8 gemäß der allgemeinen Formel (V), in der Reihenfolge von 8 nach 1,unter Verwendung von in Schritt (c) beschriebenem modifiziertem D-Lysin oder D-Omithin für Pos. 6,
(i) Abspaltens der in Schritt (h) erhaltenen Verbindung vom Träger und gegebenenfalls Aufreinigung, insbesondere durch HPLC,
(j) gegebenenfalls Umsetzens mit einer pharmazeutisch akzeptablen Säure, vorzugsweise Embonsäure.

7. Verfahren zur Herstellung einer Verbindung nach Anspruch 1, umfassend die Schritte des
(a) Ankuppelns von an der Aminogruppe mit einer Schutzgruppe versehenem D-Alanin an einen für Festphasensynthese geeigneten Träger,
(b) Abspaltens der Schutzgruppe an der Aminogruppe des Alanins,
(c) Umsetzens des an dem Harz gebundenen Alanins mit Prolin, das am Stickstoffatom mit einer Schutzgruppe versehen ist,
(d) Abspaltens der Schutzgruppe am Stickstoffatom des Prolins,
(e) Wiederholens der Schritte c) und d) mit den Aminosäuren 1 bis 8 gemäß der allgemeinen Formel (V), in der Reihenfolge von 8 nach 1,
(f) Abspaltens der im Schritt (e) erhaltenen Verbindung vom Träger,
(g) Umsetzens mit einer Carbonsäure der Formel (VII)
R⁴ - COOH (VII)
worin R⁴ wie in Anspruch 1 definiert ist,
(h) gegebenenfalls Umsetzens mit einer pharmazeutisch akzeptablen Säure, vorzugsweise Embonsäure.

8. Verfahren zur Herstellung einer Verbindung nach Anspruch 1, umfassend die Schritte des
(a) Ankuppelns von an der Aminogruppe mit einer Schutzgruppe versehenem D-Alanin an einen für Festphasensynthese geeigneten Träger,
(b) Abspaltens der Schutzgruppe an der Aminogruppe des Alanins,
(c) Umsetzens des an dem Harz gebundenen Alanins mit Prolin, das am Stickstoffatom mit einer Schutzgruppe versehen ist,
(d) Abspaltens der Schutzgruppe am Stickstoffatom des Prolins,
(e) Wiederholens der Schritte c) und d) mit den Aminosäuren 6 bis 8 gemäß der allgemeinen Formel (V), in der Reihenfolge von 8 nach 6,
(f) Abspaltens der ε-Aminoschutzgruppe von D-Lysin oder D-Omithin in Pos. 6 und Umsetzens mit einer Carbonsäure der Formel (VII)
R⁴ - COOH (VII)
worin R⁴ wie in Anspruch 1 definiert ist,
(g) Abspaltens der Schutzgruppe an der α-Aminogruppe des D-Lysins oder D-Ornithins,
(h) Wiederholens der Schritte c) und d) mit den Aminosäuren 1 bis 5 gemäß der allgemeinen Formel (IV), in der Reihenfolge von 5 nach 1,
(i) Abspaltens der in Schritt (h) erhaltenen Verbindung von dem Harz und Aufreinigung, insbesondere durch HPLC,
(j) gegebenenfalls Umsetzens mit einer pharmazeutisch akzeptablen Säure, vorzugsweise Embonsäure.

9. Verfahren nach einem der Ansprüche 6 bis 8, bei dem als Carbonsäure der allgemeinen Formel (VII) N-(4-Amidino-phenyl)-amino-4-oxo-buttersäure verwendet wird.

10. Verfahren nach einem der Ansprüche 6 bis 8, bei dem als Carbonsäure der allgemeinen Formel (VII) Imidazolidin-2-on-4-carbonsäure verwendet wird.

11. , Verfahren nach einem der Ansprüche 6 bis 10, bei dem als pharmazeutisch akzeptable Säure Embonsäure verwendet wird.

12. Verwendung der Substanzen gemäß den Ansprüchen 1 bis 4 zur Herstellung von Arzneimitteln zur Behandlung hormonabhängiger Tumore, insbesondere Prostatacarcinom oder Brustkrebs, sowie für nicht-maligne indikationen, deren Behandlung eine LH-RH-Hormonsuppression erfordert.

13. Verfahren zur Herstellung von Verbindungen gemäß den Ansprüchen 1 bis 4. umfassenden Arzneimitteln, **dadurch gekennzeichnet, daß** man eine Substanz gem. Ansprüchen 1 bis 4 mit den üblichen Träger- und Hilfsstoffen vermischt und als Arzneimittel konfektioniert.

## Claims

1. Compound of the general formula V
Ac-D-Nal(2)¹-D(pCl)Phe²-D-Pal(3)³-Ser⁴-Tyr⁵-D-Xxx⁶-Leu⁷-Arg⁸-Pro⁹-D-Ala¹⁰-NH₂ (V)
in which D-Xxx is an amino acid group of the general formula (VI) 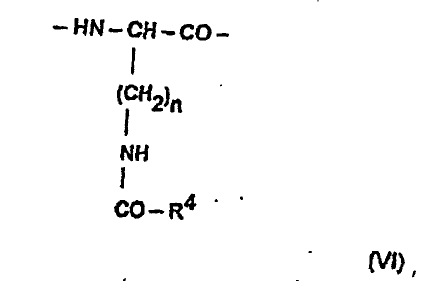 in which n is the number 3 or 4, R⁴ is a group of the formula (II) 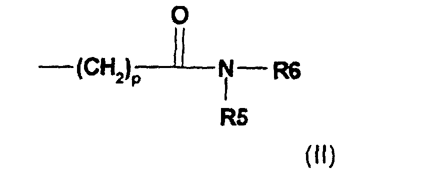 in which p is an integer from 1 to 4, R⁵ is hydrogen or an alkyl group and R⁶ is an unsubstituted or substituted aryl group or heteroaryl group,
or R⁴ is a ring of the general formula (III) 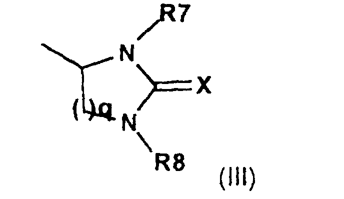 in which q is the number 1 or 2, R⁷ is a hydrogen atom or an alkyl group, R⁸ is a hydrogen atom or an alkyl group and X is an oxygen or sulphur atom, and their salts with pharmaceutically acceptable acids.

2. Compound according to Claim 1, in which Xxx is a [ε-N-4-(4-amidinophenyl)amino-1,4-dioxobutyl]lysyl group.

3. Compound according to Claim 1, in which Xxx is a [ε-N-(imidazolidin-2-on-4-yl)formyl]lysyl group.

4. Compound according to one of Claims 1 to 3, in which the salt is an embonate.

5. Pharmaceutical composition comprising a compound according to one of Claims 1 to 4.

6. Process for the preparation of a compound according to Claim 1, comprising the steps of
(a) providing the α-amino and the carboxylic acid group of D-lysine or D-ornithine with suitable protective groups,
(b) reacting the D-lysine or D-ornithine provided with protective groups with a carboxylic acid of the general formula (VII)
R⁴-COOH (VII)
in which R⁴ is as defined in Claim 1,
(c) removing the protective group on the α-carboxylic acid group of the compound obtained in step (b) for the purpose of incorporation in Pos. 6 in step (h),
(d) coupling of D-alanine provided on the amino group with a protective group to a solid support in the form of a resin,
(e) removing the protective group on the amino group of the alanine,
(f) reacting the alanine bound to the solid support with proline which is provided with a protective group on the nitrogen atom,
(g) removing the protective group on the nitrogen atom of the proline,
(h) repeating steps f) and g) with the amino acids 1 to 8 according to the general formula (V), in the sequence from 8 to 1, using modified D-lysine or D-ornithine described in step (c) for Pos. 6,
(i) removing the compound obtained in step (h) from the support and, if appropriate, purifying, in particular by HPLC,
(j) if desired, reacting with a pharmaceutically acceptable acid, preferably embonic acid.

7. Process for the preparation of a compound according to Claim 1, comprising the steps of
(a) coupling D-alanine provided with a protective group on the amino group to a support suitable for solid-phase synthesis,
(b) removing the protective group on the amino group of the alanine,
(c) reacting the alanine bound to the resin with proline which is provided with a protective group on the nitrogen atom,
(d) removing the protective group on the nitrogen atom of the proline,
(e) repeating steps c) and d) with the amino acids 1 to 8 according to the general formula (V), in the sequence from 8 to 1,
(f) removing the compound obtained in step (e) from the support,
(g) reacting with a carboxylic acid of the formula (VII)
R⁴ - COOH (VII)
in which R⁴ is as defined in Claim 1,
(h) if desired, reacting with a pharmaceutically acceptable acid, preferably embonic acid.

8. Process for the preparation of a compound according to Claim 1, comprising the steps of
a) coupling D-alanine provided with a protective group on the amino group to a support suitable for solid-phase synthesis,
(b) removing the protective group on the amino group of the alanine,
(c) reacting the alanine bound to the resin with proline which is provided with a protective group on the nitrogen atom,
(d) removing the protective group on the nitrogen atom of the proline,
(e) repeating steps c) and d) with the amino acids 6 to 8 according to the general formula (V), in the sequence from 8 to 6,
(f) removing the ε-amino protective group from D-lysine or D-ornithine in Pos. 6 and reacting with a carboxylic acid of the formula (VII),
R⁴-COOH (VII)
in which R⁴ is as defined in Claim 1,
(g) removing the protective group on the α-amino group of the D-lysine or D-ornithine,
(h) repeating steps c) and d) with the amino acids 1 to 5 according to the general formula (IV), in the sequence from 5 to 1,
(i) removing the compound obtained in step (h) from the resin and purifying it, in particular by HPLC,
(j) if desired, reacting with a pharmaceutically acceptable acid, preferably embonic acid.

9. Process according to one of Claims 6 to 8, in which N-(4-amidinophenyl)amino-4-oxobutyric acid is used as the carboxylic acid of the general formula (VII).

10. Process according to one of Claims 6 to 8, in which imidazolidin-2-one-4-carboxylic acid is used as the carboxylic acid of the general formula (VII).

11. Process according to one of Claims 6 to 10, in which embonic acid is used as the pharamceutically acceptable acid.

12. Use of the substances according to Claims 1 to 4 for the production of medicaments for the treatment of hormone-dependent tumours, in particular prostate carcinoma or breast cancer, and for non-malignant indications whose treatment necessitates LH-RH hormone suppression.

13. Process for the production of medicaments comprising compounds according to Claims 1 to 4,
**characterized in that** a substance according to Claims 1 to 4 is mixed with the customary excipients and auxiliaries and formulated as a medicament.

## Revendications

1. Composé de formule générale V
Ac-D-Nal(2)¹-D(pCl)Phe²-D-Pal(3)³-Ser⁴-Tyr⁵-D-Xxx⁶-Leu⁷-Arg⁸-Pro⁹-D-Ala¹⁰-NH₂ (V)
dans laquelle D-Xxx représente un groupe d'acide aminé de formule générale (VI) 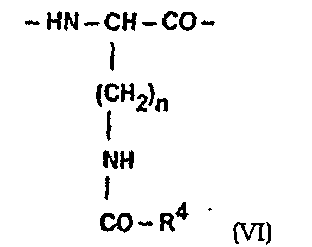 dans laquelle n est le nombre 3 ou 4, R⁴ représente un groupe de la formule (II) 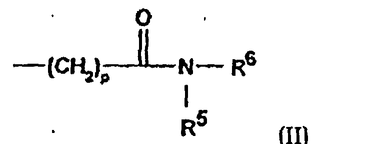 dans laquelle p est un nombre entier de 1 à 4, R⁵ signifie un atome d'hydrogène ou un groupe alkyle et R⁶ signifie un groupe aryle non substitué ou substitué ou un groupe hétéroaryle,
ou R⁴ représente un cycle de formule générale (III) 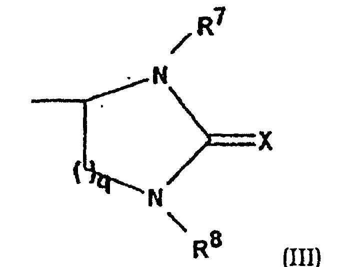 dans laquelle q est le nombre 1 ou 2, R⁷ signifie un atome d'hydrogène ou un groupe alkyle, R⁸ représente un atome d'hydrogène ou un groupe alkyle et X signifie un atome d'hydrogène ou de soufre, et leurs sels avec acides pharmaceutiquement acceptables.

2. Composé selon la revendication 1, dans lequel Xxx signifie un groupe [ε-N-4-(4-amidino-phényl)-amino-1,4-dioxo-butyl]-lysyle.

3. Composé selon la revendication 1, dans lequel Xxx signifie un groupe [ε-N-(imidazolidin-2-one-4-yl)-formyl]-lysyle.

4. Composé selon l'une des revendications 1 à 3, dans lequel le sel est un embonate.

5. Composition pharmaceutique, comprenant un composé selon l'une des revendications 1 à 4.

6. Procédé de préparation d'un composé selon la revendication 1, comprenant les étapes suivantes :
(a) on munit le groupe d'acide α-amino et d'acide carboxylique de D-lysine ou de D-omithine avec des groupes protecteurs appropriés ;
(b) on transforme la D-lysine munie des groupes protecteurs ou D-omithine avec un acide carboxylique de formule générale (VII)
R⁴-COOH (VII)
dans laquelle R⁴ est tel que défini à la revendication 1,
(c) on sépare le groupe protecteur sur le groupe acide α-carboxylique du composé obtenu à l'étape (b) à des fins d'inclusion en position 6 à l'étape (h) ;
(d) on accouple la D-alanine munie d'un groupe protecteur sur le groupe amino sur un support fixe sous forme de résine ;
(e) on sépare le groupe protecteur sur le groupe amino de l'alanine ;
(f) on transforme l'alanine liée au support fixe avec la proline, munie, sur l'atome d'azote, d'un groupe protecteur ;
(g) on sépare le groupe protecteur sur l'atome d'azote de la proline ;
(h) on répète l'étape f) et g) avec les acides aminés 1 à 8 de formule générale (V), dans l'ordre de 8 à 1, en utilisant la D-lysine modifiée décrite à l'étape (c) ou la D-omithine pour la position 6 ;
(i) on sépare le composé obtenu à l'étape (h) du support et le cas échéant on nettoie, en particulier par HPLC, ; et
(j) le cas échéant on transforme avec un acide pharmaceutiquement acceptable, de préférence l'acide embonique.

7. Procédé de préparation d'un composé selon la revendication 1, comprenant les étapes suivantes :
(a) on accouple la D-alanine munie d'un groupe protecteur sur le groupe amino sur un support approprié à la synthèse en phase solide ;
(b) on sépare le groupe protecteur sur le groupe amino de l'alanine ;
(c) on transforme l'alanine liée sur la résine avec de la proline, munie, sur l'atome d'azote, d'un groupe protecteur ;
(d) on sépare le groupe protecteur sur l'atome d'azote de la proline ;
(e) on répète l'étape c) et d) avec les acides aminés 1 à 8, de formule générale (V), dans l'ordre de 8 à 1 ;
(f) on sépare le composé obtenu à l'étape (e) du support ; et
(g) on transforme avec un acide carboxylique de formule (VII)
R⁴-COOH (VII)
dans laquelle R⁴ est tel que défini à la revendication 1,
(h) le cas échéant on transforme avec un acide pharmaceutiquement acceptable, de préférence l'acide embonique.

8. Procédé de préparation d'un composé selon la revendication 1, comprenant les étapes suivantes :
(a) on accouple la D-alanine munie d'un groupe protecteur sur le groupe amino sur un support approprié à la synthèse en phase solide ;
(b) on sépare le groupe protecteur sur le groupe amino de l'alanine ;
(c) on transforme l'alanine liée sur la résine avec de la proline, munie, sur l'atome d'azote, d'un groupe protecteur ;
(d) on sépare le groupe protecteur sur l'atome d'azote de la proline ;
(e) on répète l'étape c) et d) avec les acides aminés 6 à 8, de formule générale (V), dans l'ordre de 8 à 6 ;
(f) on sépare le groupe protecteur ε-amino de D-lysine ou D-omithine en position 6 et on transforme avec un acide carboxylique de formule (VII)
R⁴-COOH (VII)
dans laquelle R⁴ est tel que défini à la revendication 1,
(g) on sépare le groupe protecteur sur le groupe α-amino de D-Iysine ou D-omithine ;
(h) on répète l'étape c) et d) avec les acides aminés 1 à 5 de formule générale (IV), dans l'ordre de 5 à 1 ;
(i) on sépare le composé obtenu à l'étape (h) de la résine et le nettoyer, en particulier par HPLC ; et
(j) le cas échéant on transforme avec un acide pharmaceutiquement acceptable, de préférence l'acide embonique.

9. Procédé selon l'une des revendications 6 à 8,
dans lequel
comme acide carboxylique de formule générale (VII) on utilise l'acide N-(4-amidino-phényl)amino-4-oxo-butyrique.

10. Procédé selon l'une des revendications 6 à 8,
dans lequel
comme acide carboxylique de formule générale (VII) on utilise l'acide imidazolidin-2-one-4-carboxylique.

11. Procédé selon l'une des revendications 6 à 10,
dans lequel
comme acide pharmaceutiquement acceptable on utilise l'acide embonique.

12. Utilisation des substances selon les revendications 1 à 4 pour préparer des médicaments pour traiter les tumeurs hormodépendantes, en particulier le cancer de la prostate ou le cancer du sein, ainsi que pour des indications non malignes, dont le traitement exige une suppression hormonale LH-RH.

13. Procédé de préparation de composés selon les revendications 1 à 4, comprenant des médicaments,
**caractérisé en ce qu'**
on mélange une substance selon les revendications 1 à 4 avec les supports et les adjuvants habituels et qu'on en confectionne un médicament.
